# EUROPEAN PATENT APPLICATION

(11) **EP 2 985 077 A1**
(43) Date of publication of application: **17.02.2016**
(21) Application number: 14290242.8
(22) Date of filing: 11.08.2014
(51) Int. Cl.: B01J 31/16, B01J 31/22, B01J 31/12, C07C 6/04

(54) **Supported molybdenum or tungsten complexes, its preparation and use in olefin metathesis**

(71) Applicant: PFW Aroma Chemicals B.V., 3771 MT Barneveld (NL)
(72) Inventor: Mostafa, Taoufik, 690100 Villeurbanne (FR); Philipse, Hendrikus Johannes Franciskus, 6666 HT Heteren (NL); Garron, Anthony, 69170 Tarare (FR); Norsic, Sebastien, 69006 Lyon (FR); Szeto, Kai, 69100 Villeurbanne (FR); Rouge, Pascal, 69100 Villeurbanne (FR)
(74) Representative: De Kezel, Eric

(57) **Abstract**

The present invention relates to metathesis reactions of olefins, in particular in a process for manufacturing dibranched C6 olefins from isobutene, preferably for the selective synthesis of neohexene and/or 2,3-dimethylbutenes from isobutene. More particularly, the present invention relates to a supported trialkyltungsten or -molybdenum oxo complex, its preparation processes and its uses, in particular as a catalyst in metathesis or hydrogenolysis reactions of hydrocarbon compounds, more particularly as a catalyst in olefin metathesis reactions of isobutene.

## Description

The present invention relates to metathesis reactions of olefins, in particular in a process for manufacturing dibranched C6 olefins from isobutene, preferably for the selective synthesis of neohexene and/or 2,3-dimethylbutenes (2,3-dimethyl-1-butene and 2,3-dimethyl-2-butene) from isobutene. More particularly, the present invention relates to supported metal complex, its preparation processes and its uses, in particular as a catalyst in metathesis or hydrogenolysis reactions of hydrocarbon compounds, more particularly as a catalyst in olefin metathesis reactions of isobutene.

Neohexene, also called 3,3-dimethyl-1-butene, belongs to the family of the branched α-olefins (or alpha-olefins), that is to say olefins having an olefinic double bond situated on the first terminal carbon atom and possessing one or more hydrocarbon branchings. According to the definition of the prefix "neo", neohexene possesses a particular branching corresponding to the radical - C(CH₃)₃.

Neohexene conforms to the general formula

CH₂ = CH - C(CH₃)₃

Neohexene is known to possess a very high octane number, for example an RON (Research Octane Number) of 111.7, an MON (Motor Octane Number) of 93.3 (according to Internal Combustion Engines and Air Pollution, 1973, by E.F. Obert), and a relatively low vapour pressure of 48 kPa at 20 °C. For this reason, neohexene is an attractive product in the oil industry, and can in particular be used as an additive in gasolines for the motor car. In addition, neohexene can be used as an important intermediate product in the synthesis of perfume based on synthetic musk, in particular under the title "Tonalide"^{®}. 2,3-dimethylbutenes can also be used in the synthesis of "Tonalide"^{®}. Neohexene can also be used as a fungicidal agent, in particular under the title "Terbinafine"^{®}. 2,3-dimethylbut-1-ene and 2,3-dimethylbut-2-enes are useful intermediates in the manufacture of agrochemicals , flavors, cosmetics and medicines. 2,3-Dimethylbutenes and neohexene can also be alkylated to produce Triptane, also known as 2,2,3-trimethylbutane a desirable high octane component of gasoline and jet fuel as described in US 7825287 B2. Triptane is not accessible in significant proportions from refinery operated alkene/alkane isomerisation processes. It would therefore be highly desirable to develop an improved process for manufacturing dibranched hexenes namely neohexene and 2,3-dimethylbutenes.

American Patent US 3,699,035 describes a process for producing gasoline from a hexane-rich hydrocarbon feed. The process comprises averaging hexane with one or more alkanes and catalytically reforming the resulting hydrocarbons. The averaging reaction is carried out at a relatively high temperature, e.g. at about 427°C, using a catalyst mass having hydrocarbon dehydrogenation activity and olefin disproportionation activity. The catalyst mass comprises a component selected from metals of Group 8 to 10 and a component selected from Group 6. More particularly, the preferred catalyst mass consists of platinum on alumina particles mixed with tungsten oxide on silica particles.

International Patent Application WO9802244 describes a process for the metathesis of alkanes in which one or more alkanes are reacted on a solid catalyst comprising a metal hydride grafted onto a solid support. The metal of the metal hydride can be chosen from the transition metals of Groups 5 to 6 of the Periodic Table of the Elements, preferably from tantalum, tungsten and chromium. The solid support can be chosen from numerous solid oxides, preferably from silica, alumina, silica-alumina, niobium oxide and zeolites. Amongst the catalysts used for alkane metathesis, it is recommended to select tantalum hydride, tungsten hydride or chromium hydride grafted onto silica or silica-alumina. The examples show the preparations of a tantalum hydride grafted onto a silica and a tungsten hydride grafted onto a silica.

International Patent Application WO 2004/089541 describes a supported metal compound comprising a support based on aluminium oxide onto which is grafted a tungsten hydride. The support can be a mixed aluminium oxide comprising an aluminium oxide combined with at least one other oxide of an element chosen from the metals of Groups 1 to 13 and the Group 14 (with the exception of carbon) of the Periodic Table of the Elements. The other oxide can be an oxide of a metal chosen from the alkaline metals, the alkaline-earth metals, the transition metals and the elements of Groups 13 and 14 (with the exception of carbon) of said Table. More particularly, the other oxide can be an oxide of a transition metal selected from the metals of Groups 3 to 11 of said Table, in particular the elements 21 (i.e. Sc) to 29 (i.e. Cu), 39 (i.e. Y) to 47 (i.e. Ag), 57 (i.e. La) to 79 (i.e. Au) (including the lanthanides) and the actinides. Preferably, the other oxide can be an oxide of a metal chosen from the transition metals of Groups 3 to 7, the lanthanides, the actinides and the elements of Groups 13 and 14 (with the exception of carbon) of said Table. More particularly, the other oxide combined with the aluminium oxide is chosen from oxides of silicon, boron, gallium, germanium, titanium, zirconium, cerium vanadium, niobium, tantalum, chromium, molybdenum and tungsten.

International Patent Application WO 2006/013263 describes a process for metathesis of compounds containing an olefinic double bond, in particular olefins, comprising contacting said compounds with a supported metal compound comprising an aluminium oxide-based support to which a tungsten hydride is grafted.

International Patent Application WO 2006/013251 describes a process for converting ethylene into propylene, comprising contacting ethylene with a supported metal compound comprising an aluminium oxide-based support to which a tungsten hydride is grafted.

International Patent Application WO 2008/071949 describes a process for manufacturing neohexene, comprising contacting isobutene with a supported catalyst comprising a tungsten compound chosen from tungsten hydrides, organometallic tungsten compounds and organometallic tungsten hydrides, and a support comprising an oxide of aluminium, so as to form a reaction mixture comprising neohexene.

International Patent Application WO 2009/044107 describes a solid metal compound comprising (i) a solid support comprising aluminium oxide, (ii) at least one first metal compound (C1) selected from metal hydrides, organometallic compounds and organometallic hydrides, and comprising a metal (M1) selected from the lanthanides, the actinides and the metals of Groups 4 to 7 of the Periodic Table of the Elements, and (iii) at least one second metal compound (C2) comprising a metal (M2) selected from the metals of Groups 8 to 10 of said Table.

The Periodic Table of the Elements mentioned above and below is that drawn up by the IUPAC in 1991, in which the groups are numbered from 1 to 18. It is found, for instance, in "CRC Handbook of Chemistry and Physics" 76th Edition (1995-1996) by David R. Lide and published by CRC Press, Inc. (USA).

The application of homogeneous metathesis based on homogeneous catalysts generated fromWCI6 and WOCI4 as primary catalysts and SnMe4, PbMe4, Cp2TiMe2, and Cp2ZrMe2 as cocatalysts. Is described by Jiro Tsuji, Shohei Hashiguchi in Organometallics Volume 218, Issue 1, 22 September 1981, Pages 69-80. The metathesis reaction was used to convert, ethyl oleate, to Diethyl 9-octadecene-1,18-dioate which by a the Dieckmann cyclization and decarboxylation afforded civetone which can be used in perfumery. Also reported is the synthesis macrolides a class of compounds used in antibiotics. US 4515712 A describes a the use of a similar homogeneous catalyst system for the preparation of perfumes. A heterogeneous alternative to these homogeneous catalyst systems would be desirable.

The Applicants have surprisingly a different supported metal compound capable of acting as catalyst and improving catalytic reactions of saturated or unsaturated hydrocarbons. The improvements can be preferably observed in the following reactions: alkane metathesis, alkane hydrogenolysis, alkene (olefin) metathesis, alkene (olefin) oligomerisation, conversion of ethylene into propylene and more particularly conversion of isobutene into neohexene. More particularly, these reactions can be performed not only at relatively low temperatures, but also with a substantially increased catalytic activity which can be particularly more stable with time, and/or an enhanced selectivity in the hydrocarbons produced (e.g. alkanes or alkenes). These results can be obtained in catalytic reactions involving alkanes, particularly linear alkanes or preferably branched alkanes, especially branched alkanes having an "iso" structure, and also in catalytic reactions involving alkenes, e.g. olefins or alpha-olefins, particularly linear alkenes or branched alkenes e.g. having an "iso" structure.

The present invention relates to a process for manufacturing dibranched C6 olefins from isobutene which comprises the reaction of isobutene in the presence of a supported metal compound catalyst to produce dibranched C6 olefins characterized in that the said supported metal compound catalyst comprises a supported metal complex 1 and/or a supported metal complex 2 as defined hereafter.

The present invention relates to a supported metal complex 1 comprising a tungsten or molybdenum compound grafted on silica characterized in that
- the tungsten or molybdenum compound is a trialkyl-tungsten or molybdenum
   - oxo compound, and
- the tungsten or molybdenum is tethered to the silica via one oxygen atom.

A preferred solid metal complex according to the present invention can be represented by the following formula 1 wherein "M1" denotes silicon atoms which are part of the silica support, "W" denotes tungsten or molybdenum and "R" denotes a methyl alkyl residue.

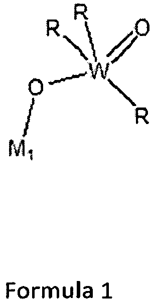

Indeed, the Applicants have unexpectedly found that this Formula 1 solid metal complex was particularly useful for the conversion of isobutene to C6 olefins as explained hereafter.

The present invention also relates to a supported metal complex 2 comprising a tungsten or molybdenum compound grafted on silica characterized in that
- the tungsten or molybdenum compound is a dialkyl-tungsten or molybdenum
   - oxo compound, and
- the tungsten or molybdenum is tethered to the silica via two oxygen atoms.

A preferred solid metal compound according to the present invention can be represented by the following formula 2 wherein "M1" and "M2" denotes silicon atoms which are part of the silica support, "W" denotes tungsten or molybdenum and "R" denotes a methyl alkyl residue.

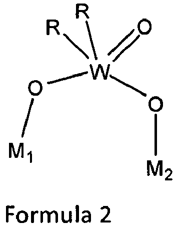

Indeed, the Applicants have unexpectedly found that this Formula 2 solid metal complex was particularly useful for the conversion of isobutene to C6 olefins as explained hereafter.

According to an embodiment of the present invention the supported metal compound catalyst comprises both supported metal complex 1 and supported metal complex 2 species as defined above.

The supported metal compound according to the present invention can advantageously be prepared according to the following consecutive synthesis steps:
0. Optional treatment of the support in order to control the hydroxyls content of the said support, said support being preferably a silica support, or silica modified with boron or aluminium.
1. Grafting an oxo-metal-ligand compound with a general formula MOX4 wherein M is W (tungsten) or Mo (molybdenum); X is any of F, Cl, Br, NRR' and OR (wherein R, R' is an alkyl and/or an aryl), (for example WOCI4) on the support (e.g. silica) to form
   a) either a metal-oxo compound wherein the metal is tethered to the support via one oxygen atom (identified hereafter as the precursor 1 of the supported metal complex 1), and/or
   b) a metal-oxo compound wherein the metal is tethered to the support via two oxygen atoms (identified hereafter as the precursor 2 of the supported metal complex 2).
2. alkylation of the compound obtained in step 1 to form
   a) either a trialkyl-metal-oxo compound wherein the metal is tethered to the support via one oxygen atom, and/or
   b) a dialkyl-metal-oxo compound wherein the metal is tethered to the silica via two oxygen atoms.

Figures 1 to 14 are illustrative embodiments of schemes according to the present invention.

Figure 1 illustrates a graphic representation of step 1 of the synthesis and of the precursor 1 of the supported metal complex 1.

Figure 2 illustrates a graphic representation of step 1 of the synthesis and of the precursor 2 of the supported metal complex 2.

Figures 3 and 4 illustrate a graphic representations of step 1 of the synthesis forming a mixture of the precursor 2 of the supported metal complex 2 and of the precursor 1 of the supported metal complex 1. The product obtained in Figure 3 denotes a mixture comprising 80mol% of the precursor 2 of the supported metal complex 2 (i.e. wherein the tungsten is tethered to the silica via two oxygen atoms) and 20mol% of the precursor 1 of the supported metal complex 1 (i.e. wherein the tungsten is tethered to the silica via one oxygen atom).

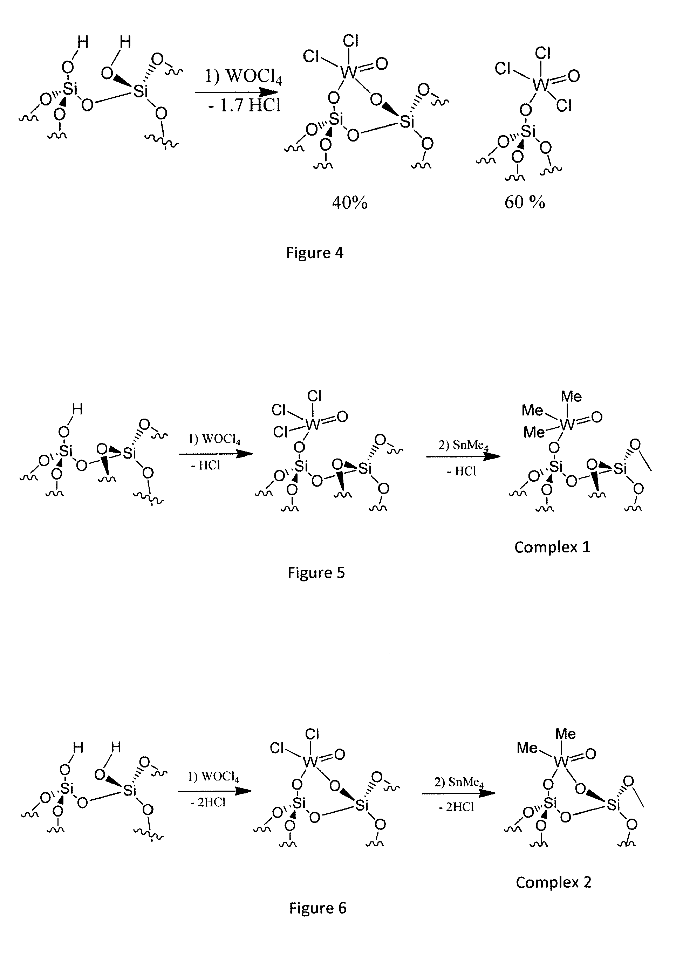

The product obtained in Figure 4 denotes a mixture comprising 40mol% of the precursor 2 of the supported metal complex 2 (i.e. wherein the tungsten is tethered to the silica via two oxygen atoms) and 60% of the precursor 1 of the supported metal complex 1 (i.e. wherein the tungsten is tethered to the silica via one oxygen atom).

Figure 5 illustrates a graphic representation of steps 1 and 2 of the synthesis and of the supported metal complex 1, i.e. the trialkyl-tungsten-oxo compound wherein the tungsten is tethered to the support via one oxygen atom (Complex 1).

Figure 6 illustrates a graphic representation of steps 1 and 2 of the synthesis and of the supported metal complex 2, i.e. the dialkyl-tungsten-oxo compound wherein the tungsten is tethered to the support via two oxygen atoms (Complex 2).

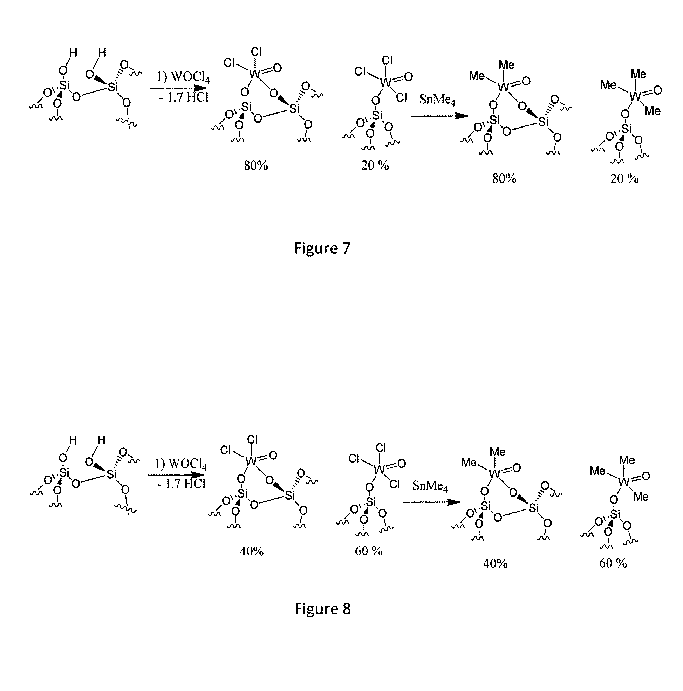

Figures 7 and 8 illustrate a graphic representations of steps 1 and 2 of the synthesis forming a mixture of the supported metal complex 2 and of the supported metal complex 1. The product obtained in Figure 7 denotes a mixture comprising 80mol% of the supported metal complex 2 (i.e. wherein the tungsten is tethered to the silica via two oxygen atoms) and 20mol% of the supported metal complex 1 (i.e. wherein the tungsten is tethered to the silica via one oxygen atom).

The product obtained in Figure 8 denotes a mixture comprising 40mol% of the supported metal complex 2 (i.e. wherein the tungsten is tethered to the silica via two oxygen atoms) and 60mol% of the supported metal complex 1 (i.e. wherein the tungsten is tethered to the silica via one oxygen atom).

Whilst not wishing to be bound by this theory, the Applicants believe that it is a combination of the properties of the support, in particular its residual hydroxyls content, together with the metal grafting step 1, in particular the temperature at which said grafting step 1 is performed, which allows to control the formation of the respective ratios between the supported metal complex 2 (i.e. wherein the tungsten is tethered to the silica via two oxygen atoms) and the supported metal complex 1 (i.e. wherein the tungsten is tethered to the silica via one oxygen atom). This control of the ratio of the supported complexes affords fine control of the catalyst activity which is beneficial in tailoring the catalytic activity to the substrate and the desired reaction. For example, higher proportion of complex 1 is desirable when weak acidity or methyl group obtained after alkylation and formation of carben is required in the catalyst i.e. dimerisation of isobutene or olefin positional isomerisation in situ. This control is illustrated in the examples. It will be apparent for the man skilled in the art that the tungsten could be replaced by molybdenum in the figures and in the above theory.

In a preferred embodiment according to the present invention, the support is silica. The support can advantageously have a specific surface area (B.E.T.) chosen from 100 to 1200 m2/g, e.g. chosen from 100 to 500 m²/g, preferably from 125 to 350 m²/g, more particularly from 150 to 250 m²/g. The specific surface area (B.E.T.) is measured according to the standard ISO 9277 (1995). The support can be predominately macroporous, microporous and/or mesoporous or a mixture thereof.

The support physically can be a powder, an extrudate or a range of catalytic shapes. The final compound is sufficiently stable to allow moulding or pelletisation of the final catalyst; during this stage a binder maybe added.

The preferred catalyst silica is generally chosen from silicon oxide substantially free from any other oxide (or containing less than 2 wt% of one or more other oxides generally present in the form of impurities).

Before use in the preparation process of the present invention the silica is preferably analyzed in order to determine its hydroxyl content which should preferably be comprised between 0.5 and 4 OH/nm, for example between 0.5 and 3.5 OH/nm2, as determined by titration and ¹H solid state NMR. In order to comply with this preferred requirement, the silica is preferably subjected to a so-called "activation" treatment which can advantageously comprise a thermal (or dehydration) treatment. The said activation treatment makes it possible to remove the water contained in the silica precursor, and also partially the hydroxyl groups, thus allowing some residual hydroxyl groups and a specific porous structure to remain. The choice of the silica precursor will preferably impact the conditions of the activation treatment, e.g. the temperature and the pressure, in order to fulfill the above final silica characteristics; this can obviously be defined on a case by case basis depending on the selection of the silica precursor and its reaction to the activation treatment. For example, the activation treatment can be carried out under a current of air or another gas, particularly an inert gas, e.g. nitrogen, as well as under reduced pressure (from low vacuum to ultra-high vacuum, preferably under high vacuum), at a temperature chosen from 50 to 1000°C, preferably from 100 to 900°C. According to an embodiment of the present invention, the synthesis of the supported metal complex 1 is favored when the hydroxyl content of the support is lower than 1.5 OH/nm2, e.g. comprised between 0.5 and 1 OH/nm2 (as determined by titration and ¹H solid state NMR).

According to another embodiment of the present invention, the synthesis of the supported metal complex 1 is favored when the support is subjected to an activation treatment as defined above at a temperature higher than 350°C, e.g. chosen from 400 to 1000°C.

According to an embodiment of the present invention, the synthesis of the supported metal complex 2 is favored when the hydroxyl content of the support is higher than 1.5 OH/nm2, e.g. comprised between 1.8 and 4 OH/nm2 (as determined by titration and ¹H solid state NMR).

According to another embodiment of the present invention, the synthesis of the supported metal complex 2 is favored when the support is subjected to a mild activation treatment as defined above at a temperature lower than 300°C, e.g. chosen from 50 to 250°C.

According to another embodiment of the present invention, the synthesis of the supported metal complexes 1 and 2 can be conducted on other oxides, for example alumina, titania, ceria, aluminosilicates, clays and mixtures of oxides. The choice of oxides for example in the case of aluminosilicates can favor the formation of complex 1 due to the spacing of the hydroxyl groups.

Suitable compounds to obtain the preferred M precursor transformable to the active catalyst after grafting and alkylation can be described by MOX4 wherein M is W or Mo, and X is any of F, Cl, Br, NRR' and OR (R and R' being an alkyl and/or an aryl).The preferred compound for proceeding with the grafting of an oxo-chloro-tungsten compound on the support is WOCI4. The preferred compound for proceeding with the grafting of an oxo-chloro-molybdenum compound on the support is MoOCI4.

The preferred compound for proceeding with the alkylation of the supported oxotungsten or oxo-molybdenum compound is tetramethyl-tin (SnMe4). Alternative alkylating compounds can also be used; for example, compounds bearing the general formulae SnR4 and/or SnR3X (with R = alkyl, benzyl derivatives bearing at least one proton in the α position (preferably substituted benzyl ligands); and X = F, Cl, Br, I, OR, NRR'). Other suitable alkylating agents may be based on Al, Ga, Si, Ge with an alkyl or aryl ligand, for example SiR4, GeR4, AIR3, GaR3, and/or AIR2X (with R = alkyl, benzyl derivatives bearing at least one proton in the α position (preferably substituted benzyl ligands); and X = F, Cl, Br, I, OR, NRR').

Whilst not wishing to be bound by this theory, the Applicants believe that this alkylation step 2, in particular this tetramethyl-tin alkylation step, which allows to control the formation of the respective ratios of C6 olefins species during the metathesis of isobutene.

The Applicants have also found that the formation of the complexes 1 and 2 by use of excess tetra-alkyl tin results in a by-product of predominately trialkyl tin chloride. Other chlorides can be produced such as dialkyl tin dichloride, alkyl tin trichloride and tin tetrachloride but these are disfavoured by the use of excess tetra-alkyl tin. The trialkyl tin chloride advantageously reacts with residual hydroxyl groups from the formation of precursors 1 and 2 to produce trialkyl tin -O- Silica (in the case of silica, other oxides such as alumina will behave in a similar manner ). Acidic functionality on supports, which is often associated with hydroxyl groups, has been implicated in the deactivation of supported metathesis catalysts, Residual hydroxyl groups on the support can also bind directly to the metathesis catalyst thereby leading to catalyst deactivation. The capping of hydroxyl groups by trialkyl tin advantageously reduces the likelihood of these no beneficial interactions.

This preparation of the catalyst in which a discrete usage of alkylation agent prior to reaction is distinctive from many other heterogeneous metathesis catalysts which employ a co-feed of alkylation agent during the metathesis reaction. In the case of tetra-alkyl tin, this has the following beneficial effects
(i) Reduced consumption of expensive reagent and generation of less waste,
(ii) No separation of toxic reagent or by-products from the metathesis product stream.

According to an embodiment of the present invention, the catalyst containing complex 1 and/or complex 2 comprises alkyl tin-O-oxide.

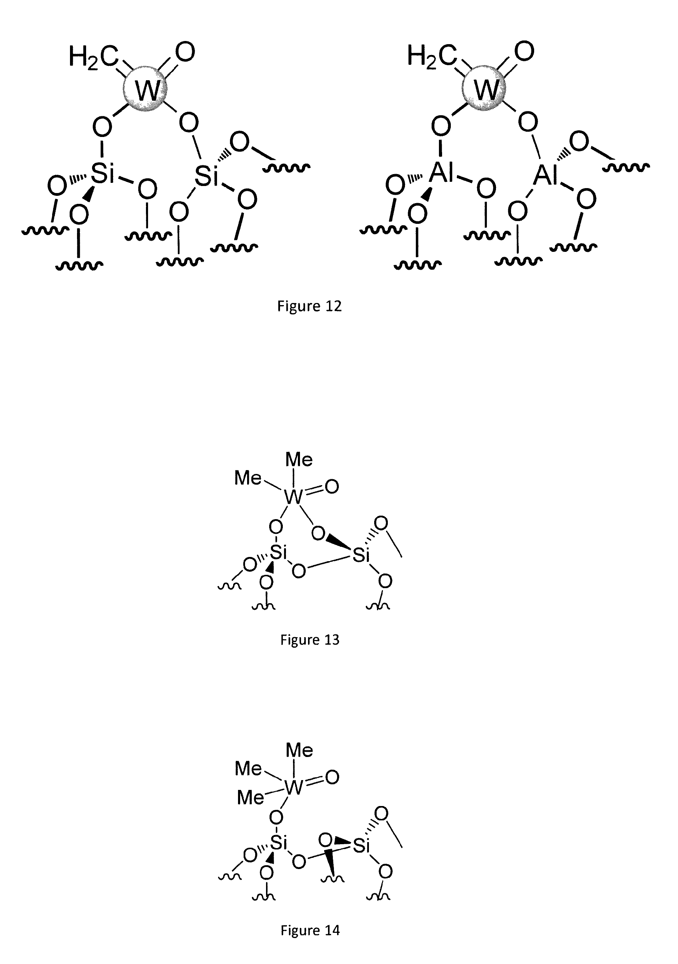

Complex 1 and complex 2 can be activated in the presence of olefins, for example isobutene, in particular propene from room temperature to e.g. 350 °C, preferably between 50-250 °C, particularly in the range of 60-180 °C. Formation of metal carbene (as graphically illustrated in figure 12 wherein two tungsten carbenes compounds corresponding to complex 2 are shown) is reflected by formation of 1 equivalent of methane during the activation of complex 1 and/or complex 2 with olefins. Activation with for example 100% 13C labelled ethene gives a signal at 200 ppm in the 13C solid state NMR spectrum, attributed to metal carbene species. Both complex types can also be activated by electromagnetic radiation, preferably radiation having energy between 2-8 eV.

The activation can thus be performed in situ - e.g. during the metathesis reaction - and or be performed beforehand.

The present invention also relates to a process for reactivating or regenerating the supported metal compound previously described, preferably after or during the use of said supported metal compound. The process can comprise contacting the supported metal compound with hydrogen or any compound capable of forming hydrogen "in situ". Hydrogen can be used alone, e.g. in a pure state, or mixed with an inert gas, e.g. nitrogen, argon or helium. The contacting can be carried out under a hydrogen absolute pressure selected from 0.01 MPa to 10 MPa, preferably from 0.1 to 2 MPa. It can be also carried out at a temperature selected from 50 to 500°C, preferably from 60 to 400°C, particularly from 70 to 350°C, especially from 80 to 300°C. The contacting can be also carried out during the use of the supported metal compound, e.g. in a process involving hydrocarbon reactions, such as the reactions subsequently described, or after and separately from said use.

The supported metal compound can also be reactivated or regenerated by direct treatment of the said alkylating agents, for example SnMe₄, from room temperature to 200 °C. Alternative reactivating and regenerating process involves treatment of the supported metal compound previously described with HCl, followed by the said alkylating agents from room temperature to 200 °C.

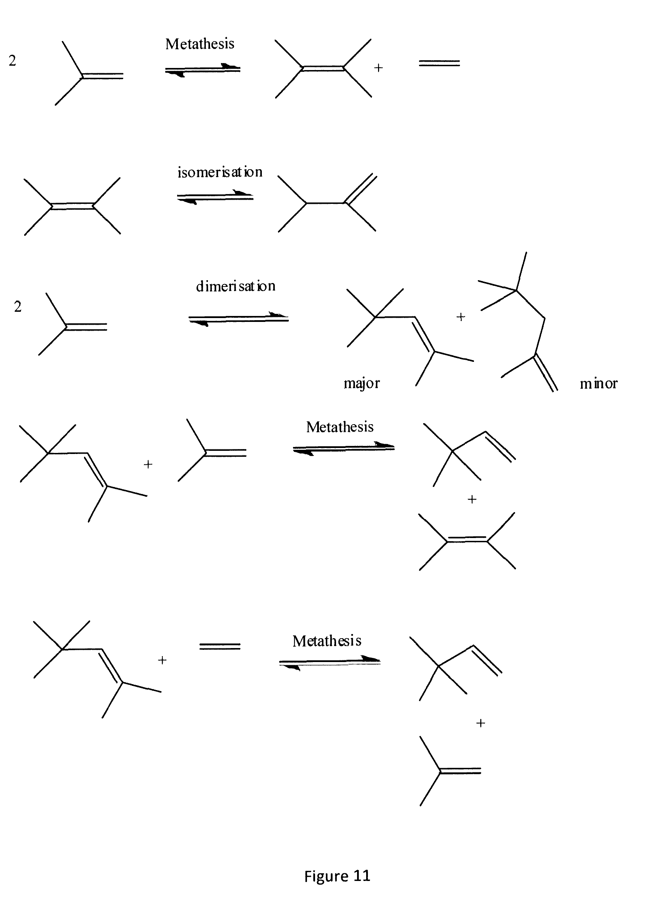

C6 branched alkenes can be obtained directly from isobutene in mild condition (150°C, 4 bars) with both high activity and selectivity. The reaction occurring consists of several reactions occurring in parallel and consecutively some of which are illustrated in figure 11.

The metathesis reactions are equilibrium limited, to one skilled in the art the yield can be effected by varying the reaction parameters such as temperature, pressure/ concentration of reactants or by continuously withdrawing a reaction product such as ethylene. It is also advantageous to withdraw ethylene continuously from the reaction mixture when 2,3-dimethylbutenes are the desired reaction product and high conversion of isobutene is desired. Practically in a continuous or semicontinuous manufacturing process it is also desirable to remove ethylene from the isobutene recycle.

Figure 11 omits secondary metathesis reactions such as the metathesis of isobutene with 2,3-dimethylbut-1-ene and 2,4,4-trimethyl pent-1-ene. These reactions can lead to loss of selectivity to the desired branched C6 olefins.

The isomerisation reaction, 2,3-dimethylbut-2-ene to 2,3-dimethylbut-1-ene, is equilibrium limited and is catalysed by acidity and/or metal complexes. When it is preferable to limit the formation of 2,3-dimethylbut-1-ene this can be achieved by reducing the amount of complex 1 in the catalyst formulation and the amount of acidity on the support by modification of the silica by boron as borates, or silianating agents ( e.g.trimethylsilyl chloride, hexamethyldisizalane).

The dimerisation of isobutene to give 2,4,4-trimethyl pent-1-ene and 2,4,4-trimethyl pent-2-ene is catalysed by acidity and/or metal complexes. The reaction is equilibrium limited and the formation of dimers is reduced by operation at high temperatures. When neohexene is a desired reaction product the yield can be increased by promoting the isobutene dimerisation reaction by adding acidity and/or a metal complex. For example by increasing the amount of complex 1 in the catalyst formulation. Alternatively since the reaction to produce neohexene requires first dimerisation of isobutene, the reaction can be conducted at longer residence times or at lower temperatures. Operation at lower temperatures can also favour neohexene formation as the metathesis reaction has a higher activation energy than the dimerisation reaction. However the dimerisation reaction is not 100% selective to the desired 2,4,4-trimethylpent-2-ene with, 2,4,4,trimethylpent-1-ene some trimers and tetramers being formed, particularly for long residence time reactions the amount of higher oligomers can increase. An alternative approach not requiring acidity and/or metal complexes active for the dimerisation is to add di-isobutenes (2,4,4trimethylpent-1-ene and 2,4,4-trimethylpent-2-ene ) preferably 2,4,4- trimethylpent-2-ene to the reaction mixture. This can be achieved by pretreating the isobutene reaction feed prior to contacting the metathesis catalyst to a dimerisation catalyst. The complete stream containing unreacted isobutene and dimers can be used as a feed for the metathesis reaction. Alternatively the di-isobutenes can be isolated, purified and added to an isobutene stream. The advantage of this is that the di-isobutenes can be manufactured under optimium conditions in a separate in an inexpensive manufacturing stage. Typically dimerisation is conducted between 60-120 °C using an acid ion-exchange resin (Mario Marchionna, Marco Di Girolamo, Renata Patrini "Light olefins dimerization to high quality gasoline components" Catalysis today, Volume 65, Issues 2-4, 20 February 2001, pages 397-403, M.D. Girolamo, L. Tagliabue "MTBE and alkylate co-production: fundamentals and operating experience", Catalysis today, Volume 52 (1999) pages 307-319.)

According to an embodiment of the present invention, the process for manufacturing neohexene and 2,3-dimethylbutenes comprises contacting of the isobutene with the catalyst. The contacting can be performed in various ways, e.g. in batch mode or preferably continuously, in a reaction zone. For example, the isobutene can be added to the catalyst, or the catalyst be added to the isobutene, or else the isobutene and the catalyst be simultaneously added into a reaction zone. The isobutene feed may optionally contain diisobutene.

The contacting can be performed at a temperature chosen in a range of from 50 to 400 °C, preferably from 70 to 300 °C, in particular from 120 to 250 °C. It can also be performed under a total absolute pressure, chosen in a range of from 0.01 to 100 MPa, preferably from 0.1 to 50 MPa, in particular from 0.1 to 30 MPa.

It can also be performed in the presence of an inert agent, either liquid or gaseous, in particular of an inert gas chosen in particular from lower alkane (e.g. isobutane, butanes, propane, ethane, methane), nitrogen, argon and helium. It can also be advantageously performed in the presence of hydrogen or of an agent forming "in situ" hydrogen, such as a cyclic hydrocarbon chosen in particular from cyclohexane, decahydronaphthalene and tetrahydronaphthalene. The hydrogen present during the contacting can in particular play the role of an agent of activation or regeneration of the catalyst. For example, the hydrogen can be used in the contacting with a hydrogen partial pressure chosen in a wide range of from 0.1 kPa to 10 MPa, preferably from 1 kPa to 1 MPa.

In addition, the contacting can be performed with quantities of isobutene and catalyst such that the molar ratio of the isobutene to the tungsten of the catalyst is chosen in a range of from 1 to 107, preferably from 2 to 105, in particular from 5 to 104. It can also be performed in a reaction zone containing the catalyst and into which the isobutene is introduced preferably continuously, and in particular with a molar rate of introduction of isobutene per mole of tungsten (or molybdenum) of the catalyst and per minute which can be chosen in a wide range of, for example, from 0.1 to 105, preferably from 1 to 105, in particular from 5 to 105.

The contacting can be performed in a gaseous phase, or in mixed gaseous/liquid phase, or in liquid phase, or else in supercritical phase, in a reaction zone adapted to the phase chosen. In particular, the contacting can be performed mainly in gaseous phase in a reaction zone, in particular under a pressure equal to or more than atmospheric pressure and less than the condensation pressure of neohexene, 2,3-dimethyl-2-butene, 2,3-dimethyl-1-butene and diisobutene.

The contacting in a mixed gaseous/liquid phase such as described above can be advantageously performed in a distillation column reactor, as described below or in American Patent US 4,242,530.

The contacting can also be performed in a supercritical phase in a reaction zone in which the catalyst is in suspension in the isobutene, in particular at a temperature higher than the critical temperature of isobutene (i.e. higher than 144 C).

It may be particularly advantageous to perform the contacting:
(i) Optionally by dimerising part of the isobutene (e.g. in a pre-reactor),
(ii) by continuously introducing the isobutene (together with the optional diisobutene from step (i) ) into a reaction zone containing the catalyst, so as to form the reaction mixture preferably continuously, and
(ii) by continuously withdrawing out of said reaction zone at least one part of the reaction mixture, so as to subject preferably continuously said part of the reaction mixture to one or more external and preferably continuous fractionation(s) outside the reaction zone, and preferably to separate and to isolate one or more component(s) of the reaction mixture, in particular the neohexene and/or the 2,3-dimethylbutenes, preferably continuously.

According to an embodiment of the present invention, the contacting can be performed in a reaction zone containing the catalyst. The catalyst is generally solid, in particular in the form of solid particles, for example in the form of a distillation packing or of a bed, through which a stream of gaseous or liquid isobutene passes, or in the form of solid particles which are maintained in suspension by the gaseous or liquid isobutene, or entrained in the form of a current with the gaseous or liquid isobutene.

The contacting can be performed in a reaction zone comprising a static reactor, a recycling reactor or a dynamic reactor. Thus, for example, the contacting can be performed in a static reactor containing fixed quantities of isobutene and catalyst introduced for a complete reaction cycle. The contacting can also be performed in a recycling reactor in which it is possible to recycle at least one of the components of the reaction mixture formed, preferably unreacted isobutene, which can be separated from the reaction mixture by a prior fractionation outside the reactor. The contacting can also be performed in a dynamic reactor in which a stream of gaseous or liquid isobutene passes into or through a bed comprising the catalyst.

In practice, the contacting can be performed in a reaction zone comprising a reactor chosen from tubular (or multi-tubular) reactors, distillation column reactors, slurry reactors, fluidised bed reactors, mechanically agitated bed reactors, fluidised and mechanically agitated bed reactors, fixed bed reactors and circulating bed reactors. The generally solid catalyst, in particular in particle form, can be arranged inside the tube(s) of a tubular (or multi-tubular) reactor. Thus, the isobutene introduced, preferably continuously, into the tube(s) can pass through it (or them) in the form of a stream and thus be contacted with the catalyst, so as to form the reaction mixture. The catalyst can also be arranged inside a distillation reactor, wherein the catalyst is preferably a component of a distillation system functioning as both a catalyst and a distillation packing, i.e. a packing for a distillation column having both a distillation function and a catalytic function: for example, rings, saddles, granulates, sheets, tubes, spirals, packed in bags, as described in American Patent US 4,242,530. The catalyst can also form the bed of a fluidised and/or mechanically agitated bed reactor, of a fixed bed reactor, or of a circulating bed reactor. The catalyst can be used in one said reactors, optionally in mixture with at least one inert solid agent, preferably chosen from silicon carbide, carbon, silicas, aluminas, silica-aluminas and aluminium silicates. The isobutene can be introduced into one of said reactors preferably continuously, and generally can pass or circulate preferably continuously in the form of a gaseous or liquid stream into the tube(s), or through the bed or the distillation packing of said reactors. In order to promote the development of the reaction towards an optimum production of dibranched C6 olefins, the process can be advantageously performed by withdrawing preferably continuously one or more component(s) of the reaction mixture, preferably the dibranched C6 olefins.

Preferably, the process also comprises separating the dibranched C6 olefins from the reaction mixture, so as to isolate the dibranched C6 olefins. The separation can be performed in various ways, discontinuously or preferably continuously. It can comprise one or more fractionation(s) of the reaction mixture, of an identical or different type, and preferably chosen from:
- fractionation by change of physical state, preferably by change of gaseous/liquid phase, in particular by distillation or by condensation,
- fractionation by molecular filtration, preferably by means of semi-permeable and selective membrane, and
- fractionation by adsorption, preferably by means of molecular sieve.

The separation can be performed in at least one fractionation zone which is either distinct and separate from the reaction zone, e.g. in one or more distillation/condensation column(s) or tower(s), or arranged in a part of said reaction zone, e.g. in a distillation column reactor, such as described previously and in American Patent US 4,242,530.

The separation can also be performed by a double (or multiple) fractionation of the reaction mixture, comprising a combination of two (or more) successive fractionations, in particular of an identical or different type, preferably chosen from the three above-mentioned types of fractionation. More particularly, the separation can be performed in two (or more) successive fractionation zones, e.g. either at least one fractionation zone arranged inside the reaction zone and the other one(s) outside said reaction zone, e.g. successively in a distillation column reactor and then in at least one distillation/condensation column or tower, or all of them arranged outside the reaction zone, e.g. in two or more distillation/condensation columns or towers, so as in particular preferably to separate and to isolate the neohexene and at least one of the other components of the reaction mixture, preferably the 2,3-dimethyl-2-butene, the 2,3-dimethyl-1-butene and/or the unreacted isobutene which is then preferably recycled into the contacting with the catalyst.

The process of the invention can be advantageously performed by means of a distillation column reactor, as described above, comprising both a reaction zone and a fractionation zone. In the reaction zone of said reactor, the isobutene can be contacted with the catalyst, preferably continuously, so as to form the reaction mixture preferably in the form of a mixed gaseous/liquid phase, and simultaneously in the fractionation zone of said reactor, one or more component(s) of said reaction mixture is (are) preferably continuously separated, in particular ethylene, unreacted isobutene and preferably dibranched C6 olefins, so as particularly to isolate the dibranched C6 olefins and more particularly the unreacted isobutene which is then preferably recycled into said reaction zone.

The fractionation by change of the physical state, preferably of the gaseous/liquid phase of the reaction mixture, can be performed in one or more distillation/condensation columns or towers, or else in a distillation column reactor, in particular by cooling or heating the reaction mixture, so as to condense or to vaporise at least one of the components of the reaction mixture, preferably dibranched C6 olefins, and preferably to separate them and to isolate them from the rest of the mixture. The fractionation can in particular be performed by distillation of the reaction mixture, more particularly in at least one distillation column or one distillation column reactor, so as to withdraw at the top of said column or reactor ethylene, then at a lower level unreacted isobutene, which is preferably recycled into the reaction zone, and at a still lower level neohexene, which can thus be separated and isolated in its turn from the rest of the liquid mixture containing in particular 2,3-dimethyl-2-butene, 2,3-dimethyl-1-butene and diisobutene. The rest of the liquid mixture can be then subjected to a fractionation in its turn, in particular by distillation, so as to separate and to isolate the 2,3-dimethyl-2-butene and/or the 2,3-dimethyl-1-butene from the diisobutene, thus upgrading the process, since 2,3-dimethyl-2-butene and/or 2,3-dimethyl-1-butene are sought-after products because of their relatively high octane number.

The fractionation by molecular filtration can be performed by passing the reaction mixture through one or more semi-permeable and selective membrane(s), so as to arrest at least one of the components of the reaction mixture, preferably the neohexene, and to allow the rest of the reaction mixture, in particular the ethylene, the 2,3-dimethyl-2-butene, the 2,3-dimethyl-1-butene, the diisobutene and optionally the unreacted isobutene, to pass through.

The fractionation by adsorption can be performed by passing the reaction mixture onto one or more molecular sieves, so as to retain at least one of the components of the reaction mixture, preferably the neohexene, and to allow the other component(s) of the reaction mixture, in particular the ethylene, the 2,3-dimethyl-2-butene, the 2,3-dimethyl-1-butene, the diisobutene and optionally the unreacted isobutene, to pass through, and by subjecting the component(s) of the reaction mixture thus retained to at least one desorption step, preferably by the TSA method ("Temperature Swing Adsorption") or the PSA method ("Pressure Swing Adsorption"), so as to isolate it (or them).

The separation can also comprise a combination of at least two or three successive fractionations of a different type, preferably chosen from the three above-mentioned fractionations, so as in particular preferably to separate (a) neohexene and at least one of the following other components of the reaction mixture: (b) 2,3-dimethyl-1-butene, (c) 2,3-dimethyl-2-butene, (d) diisobutene, (e) ethylene and (f) unreacted isobutene.

The process can advantageously comprise separating unreacted isobutene from the reaction mixture, and then preferably recycling it into the contacting with the catalyst, so as in particular to increase the output of the process. The process can also comprise separating 2,3-dimethyl-1-butene and/or 2,3-dimethyl-2-butene from the reaction mixture, and preferably isolating it (or them), in particular by reason of its (their) relatively high octane number.

The process can advantageously comprise separating and isolating the C5+ hydrocarbon products (i.e. comprising at least 5 carbon atoms), e.g. C5+ olefins and optionally C5+ alkane(s), including neohexene, from the reaction mixture as a single component, so as in particular to blend said single component with gasoline to enhance the gasoline octane number, or in particular to use said single component as a gasoline blendstock.

The process can also advantageously comprise separating the C5+ hydrocarbon products, e.g. C5+ olefins and optionally C5+ alkane(s), including neohexene, from the reaction mixture as a single component, followed by separating and isolating at least one separated fraction from said single component, so as in particular to blend said at least one separated fraction with gasoline to enhance the gasoline octane number, or in particular to use said at least one separated fraction as a gasoline blendstock.

The present invention also relates to the use of the at least one previously mentioned separated fraction, for blending it with gasoline to enhance the gasoline octane number. It also relates to the use of the at least one previously mentioned separated fraction as a gasoline blendstock.

The process of the invention is also particularly advantageous for manufacturing dibranched C6 olefins, namely in a single (reaction) stage and with a relatively high specificity.

The present invention further relates to the use of the supported metal compound previously described, essentially as a catalyst, in a process involving hydrocarbon reactions, preferably comprising contacting said supported metal compound with one or more starting hydrocarbons optionally in the presence of hydrogen, and involving the splitting and recombining of carbon-carbon and/or carbon-hydrogen and/or carbon-metal bonds, so as to produce final hydrocarbon(s) different from the starting hydrocarbons.

More particularly, the present invention relates to the use of the supported metal compound, essentially as a catalyst, in a process for manufacturing saturated or unsaturated hydrocarbon(s) having a modified carbon skeleton. The process preferably comprises contacting at least one saturated or unsaturated aliphatic hydrocarbon with itself, or with at least one other saturated or unsaturated aliphatic hydrocarbon, or with at least one aromatic hydrocarbon substituted by at least one hydrocarbon radical, or else with at least one saturated or unsaturated alicyclic hydrocarbon substituted by at least one hydrocarbon radical, e.g. an alkyl, alkenyl or alkynyl radical, in the presence of said supported metal compound and optionally of hydrogen. The hydrocarbons used in these processes can be saturated or unsaturated hydrocarbons, preferably selected from alkanes and alkenes (olefins). More particularly, the hydrocarbons involved in these processes can be chosen from:
- linear aliphatic saturated or unsaturated hydrocarbons, in particular from C₂ to C₃₀, preferably from C₂ to C₂₀,
- branched aliphatic saturated or unsaturated hydrocarbons, in particular from C₄ to C₃₀, preferably from C₄ to C₂₀, especially having an "iso" structure,
- aromatic hydrocarbons substituted by at least one hydrocarbon radical and particularly selected from C₇ to C₃₀, preferably from C₇ to C₂₀, said hydrocarbon radical preferably being linear or branched and selected from C₁ to C₂₄, preferably from C₁ to C₁₄,
- alicyclic saturated or unsaturated hydrocarbons substituted by at least one hydrocarbon radical and particularly selected from C₄ to C₃₀, preferably from C₄ to C₂₀, said hydrocarbon radical preferably being linear or branched and selected from C₁ to C₂₇, preferably from C₁ to C₁₇.

More particularly, the supported metal compound can be used, essentially as a catalyst, in a process comprising alkane metathesis reactions, preferably metathesis reactions of an alkane with itself, i.e. alkane self-metathesis reactions, or metathesis reactions of an alkane with at least one other alkane, i.e. alkane cross-metathesis reactions. In particular, the alkane(s) can be either linear or preferably branched especially with an "iso" structure, and selected from the C₂ to C₁₀ alkanes, particularly from ethane, propane, n-butane, isobutane, n-pentane, isopentane, neopentane, n-hexane, isohexane, neohexane, n-heptane, isoheptane, neoheptane, n-octane, isooctane, neooctane, n-nonane, isononane, neononane, n-decane, isodecane and neodecane. The supported metal can be also used in a specific process converting isobutane into 2,3-dimethylbutane. Generally these processes can be carried out at a temperature selected from 20 to 500°C, preferably from 50 to 400°C, particularly from 50 to 350°C, under an absolute pressure particularly selected from 0.1 to 100 MPa, preferably from 0.1 to 50 MPa. The supported metal compound can be particularly used in the processes as described in International Patent Application WO 98/02244.

The supported metal compound can be also used, essentially as a catalyst, in a process comprising alkene (olefin) metathesis reactions, preferably metathesis reactions of an alkene with itself, i.e. alkene self-metathesis reactions, or metathesis reactions of an alkene with at least one other alkene, i.e. alkene cross-metathesis reactions, the alkene(s) being preferably linear or branched especially with an "iso" structure, and selected from the C₂ to C₁₀ alkenes, e.g. from α-olefins (alpha-olefins), particularly from ethylene, propylene, 1-butene, isobutene, trans-2-butene, cis-2-butene, 1-pentene, 2-pentene, isopentene,1-hexene, 2-hexene, 3-hexene, isohexene, neohexene, 1-heptene, 2-heptene, 3-heptene, isoheptene, neoheptene, 1-octene, 2-octene, 3-octene, 4-octene, isooctene, neooctene, 1-nonene, 2-nonene, 3-nonene, 4-nonene, isononene, neononene, 1-decene, 2-decene, 3-decene, 4-decene, 5-decene, neodecene and isodecene; and/or cyclic alkenes, for example cyclohexene, cyclooctene, 1,5-cyclooctadiene, cyclododecene, cyclododecadiene and 1,5,9-cyclododecatriene, cyclohexadecene, 1,9-cyclohexadecadiene, 1,5,9,13-cyclohexadecatetraene. The supported metal compound can be used in a specific process converting ethylene into propylene as described in International Patent Application WO 2006/013251, or in a process comprising alkene (olefin) metathesis reactions as described in International Patent Application WO 2006/013263. It can be also used in a specific process converting isobutene into neohexene (i.e. 3,3-dimethyl-1-butene).

The supported metal compound can be also be used in self and cross metathesis reactions of unsaturated fatty acid esters as described in J. Braz. Chem. Soc., Vol. 9, No. 1, 1-11, 1998.

The supported metal compound can be also used, essentially as a catalyst, in a process comprising alkene (olefin) oligomerisation, preferably ethylene or propylene oligomerisation.

The supported metal compound can be also used, essentially as a catalyst, in a process comprising a non-oxidative coupling reaction of methane, preferably for producing ethane. The process can be carried out at a temperature selected from 20 to 500°C, preferably from 50 to 400°C, particularly from 70 to 350°C, and preferably under an absolute pressure selected from 0.01 to 100 MPa, preferably from 0.1 to 50 MPa. The supported metal compound can be particularly used in the process as described in International Patent Application WO 03/104171.

The supported metal compound can be also used, essentially as a catalyst, in a process comprising a methane-olysis reaction preferably carried out by contacting methane with at least one other aliphatic hydrocarbon, or with at least one aromatic or alicyclic hydrocarbon substituted by at least one hydrocarbon radical. More particularly, the process can comprise contacting, in the presence of the supported metal compound, methane with:
- at least one other aliphatic hydrocarbon chosen from linear aliphatic hydrocarbons particularly from C₂ to C₃₀, preferably from C₃ to C₂₀, and from branched aliphatic hydrocarbons particularly from C₄ to C₃₀, preferably from C₄ to C₂₀, especially having an "iso" structure, or
- at least one aromatic hydrocarbon substituted by at least one hydrocarbon radical and particularly selected from C₇ to C₃₀, preferably from C₇ to C₂₀, said hydrocarbon radical preferably being linear or branched, saturated or unsaturated, in particular from C₁ to C₂₄, preferably from C₁ to C₁₄, e.g. an alkyl, alkenyl or alkynyl radical, or
- at least one alicyclic hydrocarbon substituted by at least one hydrocarbon radical and particularly selected from C₄ to C₃₀, preferably from C₄ to C₂₀, said hydrocarbon radical preferably being linear or branched, saturated or unsaturated, in particular from C₁ to C₂₇, preferably from C₁ to C₁₇, e.g. an alkyl, alkenyl or alkynyl radical.

The supported metal compound can be also used in a process comprising methane-olysis reactions preferably carried out by contacting methane with natural gas, liquefied petroleum gas or LPG, wet gas or wet natural gas (i.e. a mixture of methane with alkanes of C₂ to C₅, or C₃ and/or C₄), natural-gas liquid or NGL, or cuts of light hydrocarbons preferably selected from cuts from C₁ to C₆, from C₁ to C₅, from C₁ to C₄, from C₁ to C₃, and from C₁ to C₂.

The supported metal compound can be used in such processes comprising a methane-olysis reaction generally carried out at a temperature selected from 20 to 500°C, preferably from 50 to 400°C, particularly from 70 to 350°C, and preferably at a methane partial pressure selected from 0.1 to 100 MPa, particularly from 0.1 to 50 MPa. The supported metal compound can be particularly used in the methane-olysis processes as described in International Patent Applications WO 01/004077 and WO 03/066552.

The supported metal compound can be also used in a process comprising a cross-metathesis reaction between said supported metal compound and at least one starting hydrocarbon preferably selected from:
- linear or branched aliphatic hydrocarbons, in particular from C₂ to C₃₀, preferably from C₂ to C₂₀, especially having an "iso" structure, or
- alicyclic hydrocarbons substituted by at least one hydrocarbon radical and particularly selected from C₄ to C₃₀, preferably from C₄ to C₂₀, said hydrocarbon radical being linear or branched, in particular from C₁ to C₂₇, preferably from C₁ to C₁₇, e.g. an alkyl, alkenyl or alkynyl radical.

The supported metal compound can be used in such a cross-metathesis process carried out at a temperature selected from 20 to 500°C, preferably from 50 to 400°C, especially from 70 to 350°C, and preferably under an absolute pressure selected from 0.01 to 50 MPa, preferably from 50 to 20 MPa. It can be particularly used in a cross-metathesis process as described in International Patent Application WO 00/027781.

The supported metal compound can be also used in a process comprising a hydrocarbon hydrogenolysis reaction. Generally, a hydrogenolysis reaction comprises contacting at least one starting hydrocarbon with hydrogen in the presence of the supported metal compound, so as to produce at least one final hydrocarbon having a carbon skeleton modified. The starting hydrocarbon can be selected (i) from saturated hydrocarbons, e.g. the above-mentioned alkanes, (ii) from hydrocarbon polymers or oligomers, e.g. (co)polymers or (co)oligomers of one or more olefinic or vinyl monomers, in particular polyolefins, preferably polyethylene, polypropylene, poly-1-butene, polyisobutene, copolymers of ethylene with at least one alpha-olefin from C₃ to C₈, copolymers of propylene with at least one alpha-olefin from C₄ to C₈, and copolymers of isobutene with 1-butene, or aromatic polyvinyl, preferably polystyrene or polyalphamethylstyrene, or (iii) from hydrocarbon waxes, e.g. Fisher-Tropsch waxes, polyethylene waxes and polypropylene waxes. Generally, such processes can be carried out at a temperature selected from 20 to less than 400°C, preferably from 50 to 300°C, preferably under a hydrogen partial pressure selected from 10⁻³ to 20 MPa, preferably from 10⁻² to 10 MPa, in particular for a time selected from 5 minutes to 100 hours, preferably from 10 minutes to 50 hours. The supported metal compound can be particularly used in the process as described in European Patent Application EP 0 840 771.

The following examples illustrate the present invention.

### Examples

### Example 1:

Preparation of SiO2-700 Silica Aerosil from Degussa with a specific area of 200 m2/g was calcined at 500 °C for 4 h then dehydroxylated at 700 °C for 12 h under high vacuum (10-5 mmHg). After this treatment, the specific area was about 200 m2/g with an OH density of 0.85 OH/nm2, determined by titration and 1H NMR. (0.27 mmol OH/g on SiO2-700)

### Example 2:

Preparation of SiO2-500: Silica Aerosil from Degussa with a specific area of 200 m2/g was dehydroxylated at 500 °C for 12 h under high vacuum (10-5 mmHg). After this treatment, the specific area was about 200 m2/g with an OH density of 1.2 OH/nm2, determined by titration and 1H NMR. (0.40 mmol OH/g on SiO2-200)

### Example 3:

Preparation of SiO2-200: Silica Aerosil from Degussa with a specific area of 200 m2/g was dehydroxylated at 200 °C for 12 h under high vacuum (10-5 mmHg). After this treatment, the specific area was about 200 m2/g with an OH density of 2.35 OH/nm2, determined by titration and 1H NMR. (0.780 mmol OH/g on SiO2-200)

### Example 4:

Preparation of SiO2-150: Silica Aerosil from Degussa with a specific area of 200 m2/g was dehydroxylated at 150 °C for 12 h under high vacuum (10-5 mmHg). After this treatment, the specific area was about 200 m2/g with an OH density of 2.69 OH/nm2, determined by titration and 1H NMR. (0.890 mmol OH/g on SiO2-150)

### Example 5:

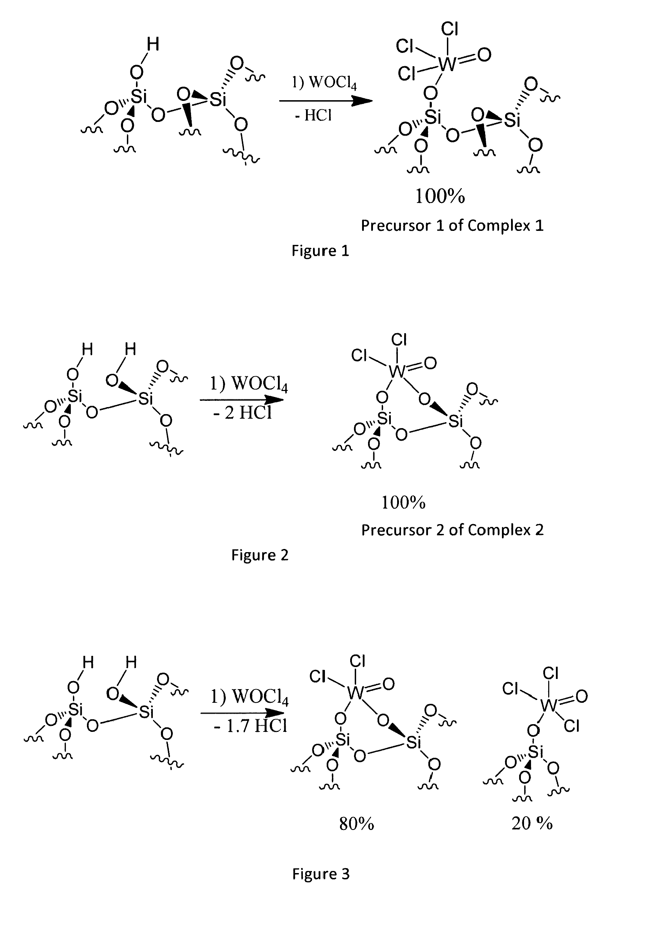

Synthesis of catalyst WOCI4 supported on SiO2-150 (precursor 2 of the complex 2 - as illustrated graphically in figure 2)
1 g of the SiO02-150, was contacted with oxotetrachlorotungsten (W(=O)CI4) (160 mg), and stirred at 80 °C during 4h in a double-Schlenck (this step can be performed either in toluene and/or by mechanical mixture without solvent). All volatile compounds were condensed into an infrared cell (of known volume) in order to quantify HCl evolved during grafting by infrared spectroscopy. The infrared analysis showed that 1.7 HCl/W are released during the grafting. Freshly distilled toluene was then added and the solid was washed three times. The resulting powder was dried under vacuum and stored in glovebox at -30 °C. Elemental analysis showed that we have on the catalyst 6.5 wt% of W and 2.5 wt% of Cl, which gives a ratio of 2 Cl/W.

### Example 6:

Synthesis of catalyst WOCI4 supported on SiO2-200 (mixture of precursor 2 of the complex 2 and of precursor 1 of the complex 1- as illustrated graphically in figure 3)
1 g of the SiO2-700, was contacted with oxotetrachlorotungsten (W(=O)CI4) (160 mg), and stirred at 80 °C during 4h in a double-Schlenck (this step can be performed either in toluene and/or by mechanical mixture without solvent). All volatile compounds were condensed into an infrared cell (of known volume) in order to quantify HCl evolved during grafting by infrared spectroscopy. The infrared analysis showed that 1.7 HCl/W are released during the grafting. Freshly distilled toluene was then added and the solid was washed three times. The resulting powder was dried under vacuum and stored in glovebox at -30 °C. Elemental analysis showed that we have on the catalyst 7.26 wt% of W and 3.13 wt% of Cl, which gives a ratio of 2.2 Cl/W.

### Example 7:

Synthesis of catalyst WOCI4 supported on SiO2-500 (mixture of precursor 2 of the complex 2 and of precursor 1 of the complex 1- as illustrated graphically in figure 4)
1 g of the SiO2-700, was contacted with oxotetrachlorotungsten (W(=O)Cl4) (150 mg) and stirred at 80 °C during 4h in a double-Schlenck (this step can be performed either in toluene and/or by mechanical mixture without solvent). All volatile compounds were condensed into an infrared cell (of known volume) in order to quantify HCl evolved during grafting by infrared spectroscopy. The infrared analysis showed that 1.2 HCl/W are released during the grafting. Freshly distilled toluene was then added and the solid was washed three times. The resulting powder was dried under vacuum and stored in glovebox at -30 °C. Elemental analysis showed that we have on the catalyst 5.6 wt% of W and 2.88 wt% of Cl, giving a ratio of 2.7 Cl/W.

### Example 8:

Synthesis of catalyst WOCI4 supported on SiO2-70 (precursor 1 of the complex 1 - as illustrated graphically in figure 1)
2 g of the material 1, was contacted with oxotetrachlorotungsten (W(=O)CI4) (120 mg) in toluene, and stirred at 80 °C during 4h in a double-Schlenck (this step can be performed either in toluene and/or by mechanical mixture without solvent). All volatile compounds were condensed into an infrared cell (of known volume) in order to quantify HCl evolved during grafting by infrared spectroscopy. The infrared analysis showed that 0.9 HCl/W are released during the grafting. Freshly distilled toluene was then added and the solid was washed three times. The resulting powder was dried under vacuum and stored in glovebox at -30 °C. Elemental analysis showed that we have on the catalyst 4.5 wt% of W and 1.66 wt% of Cl, giving a ratio of 3.1 Cl/W.

### Example 9:

Alkylation of supported WOCI4 on SiO2-150°C (precursor 2 as illustrated in figure 2) by SnMe4 and synthesis of the complex 2 (as illustrated graphically in figure 6).

100 % ¹³C labeled *Me4Sn was prepared by the alkylation of SnCl4 using ¹³CH₃MgCl in n-buthylether.

The reaction of solid precursor 1 of the complex 2 (as illustrated graphically in figure 2) with a vapor pressure of *Me₄Sn was performed in gas phase at 80°C during 12h. All volatiles were transferred to an NMR Young Tube containing C₆D₆, the resulting ¹³C displayed SnMe₄, as reflected by the methyl carbon at -9.43 ppm with a J(117/119Sn-CH) of 320.4/335.1 Hz. The rest of the major signal can easily be attributed to the sole and expected Me3SnCl side product, whose methyl carbon is visible at -1,74 ppm with a J(¹¹⁷/¹¹⁹Sn-CH) of ^{360.1}/^{377.4} Hz.^{59,60}

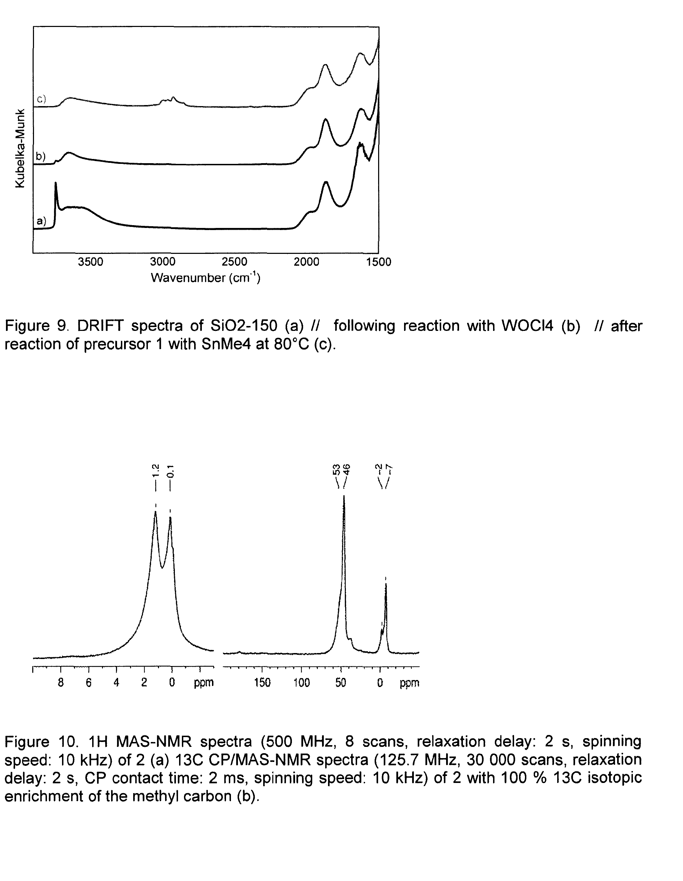

The DRIFT spectra concerning the preparation of the complex 2 are displayed in Figure 9. New peaks compared to its precursor 1 appeared. Bands at 1455 and 1490 cm-1, concomitant with the bands in the 2800 to 3000 cm-1 region are characteristic of δ(C-H) and v(C-H) respectively, for methyl moiety. Moreover, the small amount of remained isolated silanol group is fully consumed after the alkylation. The ¹H MAS-NMR spectrum of complex 2 (Figure 10, a) shows two broad signals around 1.2 and 0.1 ppm, from the methyls moieties from tungsten and tin respectively. Concomitant with the presence of a sharp and intense signal at 46 ppm in the ¹³C CP/MAS spectrum (Figure 3, b), assigned to the characteristic W-Me fragment of (≡SiO)₂W(=O)Me₂, as observed in the closely related [(t-Bu)3SiO]2W(=O)Me2 soluble model. In addition, peaks at -7 and -2 ppm are attributed to methyl attached to Sn fragment on silica Formation of surface tin methyl species is due probably to reaction of SnMe4 or SnMe₃Cl (formed during the alkylation) with the remaining free silanol groups on the surface. EXAFS data is consistent with the previously described data from DRIFT and solid state NMR. The extracted data fitted to the k2.χ(k) and k3.χ(k) (Table 1) are equal to the tungsten surface species depicted in Figure 7.

**Table 1. EXAFS parameters for the supported complex resulting from the reaction of [(≡SiO)₂W(=O)Cl₂] with SnMe₄,**

| Type of neighbor | Number neighbors | of Distance (Å) | σ² (Å²) |
|---|---|---|---|
| W=O | 1.0 | 1.70(1) | 0.0017(8) |
| W-OSi≡ | 1.8(2) | 1.88(1) | 0.0014(5) |
| W-CH₃ | 1.9(4)^{b} | 2.12(2) | 0.006(4) |
| W-Cl | 0.3(2) | 2.36(2) | 0.002(2) |
| W--O(Si≡)₂ | 1.0 | 2.91(3) | 0.002(2) |
| The errors generated by the EXAFS fitting program "RoundMidnight" are indicated in parentheses. ^{a} Δk: [2.2 - 18.0 Å⁻¹] - ΔR: [0.7-2.9 Å] ([0.7-2.4 Å], when considering only the first coordination sphere without Cl); S₀² = 0.94; ΔE₀ = 8.7 ± 2.0 eV (the same for all shells); Fit residue: ρ = 10.5 %; Quality factor: (Δχ)²/ν = 3.81, with ν = 11 / 24 ([(Δχ)²/ν]₁ = 4.99 with ν = 11 / 19, considering only three backscatterers, oxo, -O and -C in the first coordination sphere of W). ^{b} Shell constrained to the parameters above and below. | | | |

### Example 10 :

Alkylation of supported WOCI4 on SiO2-200°C (mixture of precursors 1 and 2 as illustrated in figure 3) by SnMe4 and synthesis of the mixture of complex 1 and complex 2 (as illustrated graphically in figure 7).

1 g of the material (mixture of precursors 1 and 2 as illustrated in figure 3) was exposed to SnMe4 vapor pressure at 80°C.

### Example 11 :

Alkylation of supported WOCI4 on SiO2-500°C (mixture of precursors 1 and 2 as illustrated in figure 4) by SnMe4 and synthesis of the mixture of complex 1 and complex 2 (as illustrated graphically in figure 8)
1 g of the material (mixture of precursors 1 and 2 as illustrated in figure 4), was exposed to SnMe4 vapor pressure at 80°C.

### Example 12:

Alkylation of supported WOCI4 on SiO2-700°C (precursor 1 as illustrated in figure 1) by SnMe4 and synthesis of the complex 1 (as illustrated graphically in figure 5)
The reaction of solid precursor 1 of the complex 1 (as illustrated graphically in figure 1) with a vapor pressure of Me4Sn was performed in gas phase at 80°C.

### Example 13 :

### Isobutene metathesis:

The evaluation of the activity of the catalyst in isobutene metathesis has been performed in a continuous flow reactor. The catalytic test were performed at 150°C, with a flow rate of 10mL.min-1 (5 mL.min-1 isobutene + 5mL.min-1 argon), pressure = 4 bar and 0.25 g of catalyst. The catalysts were obtained according to the procedures detailed in the previous examples.

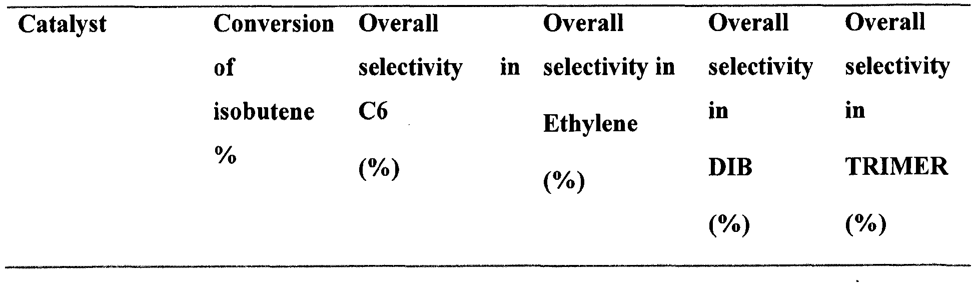

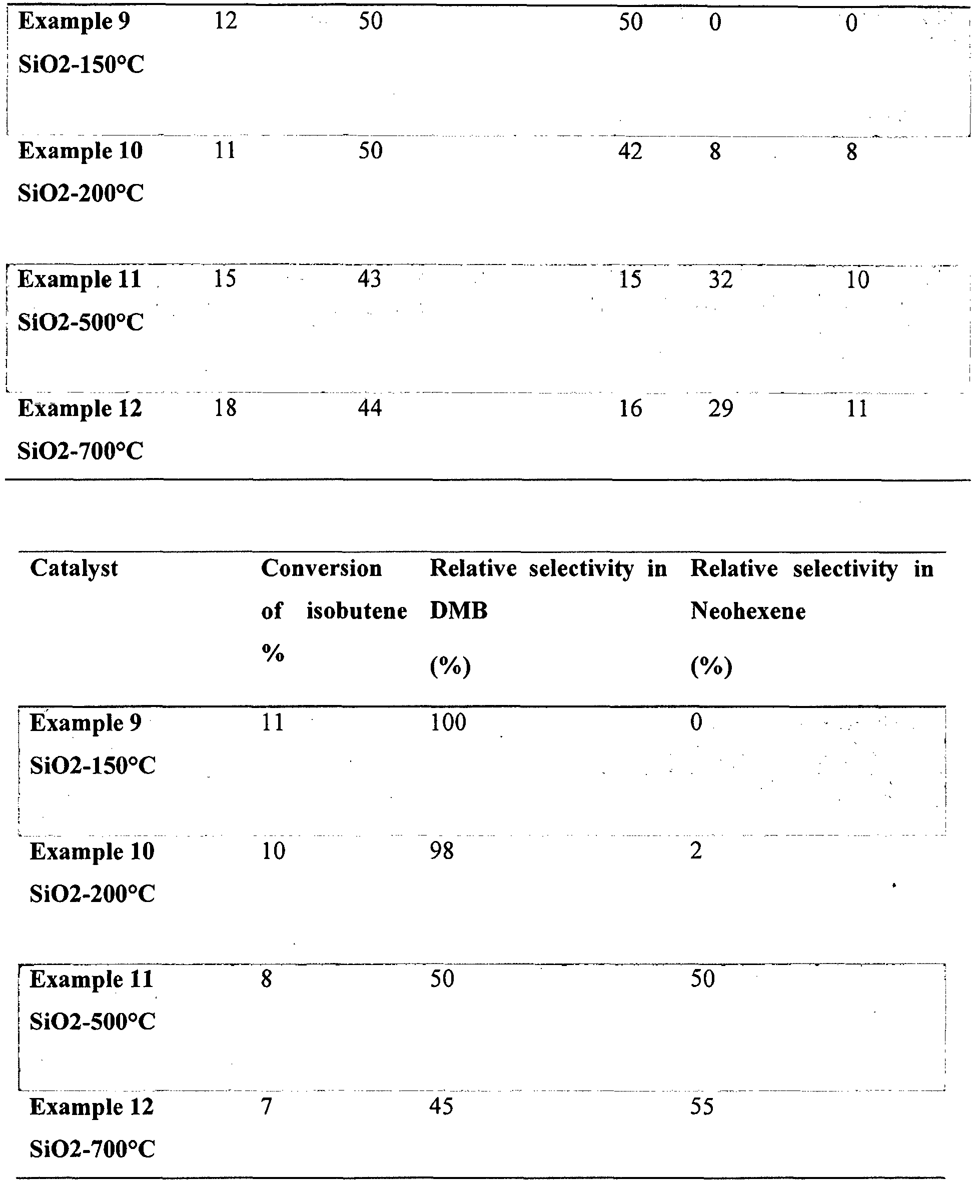

As can be seen from the results given in the table, the effect of dehydroxylation temperature is to target specific surface active species. In one hand, after a dehydroxylation at 150°C and alkylation at 80°C, the metathesis active species are 100% of di-podal tungsten dioxodimethyl (as illustrated in figure 13) which favours the formation of neohexene:

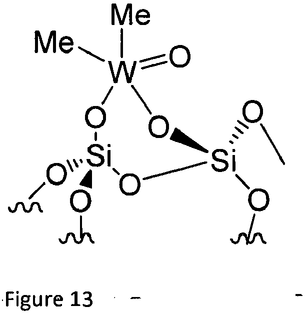

In the other hand, after a dehydroxylation at 700°C and alkylation at 80°C, the metathesis active species are 100% of mono-podal tungsten oxotrimethyl (as illustrated in figure 14) which favours the formation of DMB:

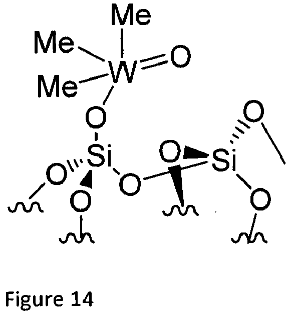

These two species and their mixtures are active for isobutene metathesis reactions.

## Claims

1. Supported metal compound catalyst for the metathesis or hydrogenolysis reactions of hydrocarbon compounds **characterized in that** the said supported metal compound catalyst comprises a supported metal complex 1 and/or a supported metal complex 2 wherein
- the supported metal complex 1 comprises a tungsten or molybdenum compound grafted on an oxide support wherein the tungsten or molybdenum compound is a trialkyl-tungsten- or molybdenum- oxo compound, and the tungsten or molybdenum is tethered to the oxide support via one oxygen atom, and
- the supported metal complex 2 comprises a tungsten or molybdenum compound grafted on an oxide support wherein the tungsten or molybdenum compound is a dialkyl-tungsten- or molybdenum- oxo compound, and the tungsten or molybdenum is tethered to the oxide support via two oxygen atoms.

2. Supported metal compound catalyst according to the preceding claim wherein the oxide support is silica.

3. Supported metal compound catalyst according to any of the preceding claims wherein the hydroxyl content of the support is comprised between 0.5 and 4 OH/nm2, as determined by titration and ¹H solid state NMR.

4. Supported metal compound catalyst according to any of the preceding claims wherein the hydroxyl content of the support is comprised between 0.5 and 1 OH/nm2 (as determined by titration and ¹H solid state NMR).

5. Supported metal compound catalyst according to any of the preceding claims wherein the hydroxyl content of the support is comprised between 1.8 and 4 OH/nm2 (as determined by titration and ¹H solid state NMR).

6. Process for the preparation of the supported metal compound according to any of the preceding claims wherein the process comprises the following consecutive synthesis steps:
0. Optional treatment of the support in order to control the hydroxyls content of the said support, said support being preferably a silica support, or silica modified with boron or aluminium support;
1. Grafting an oxo-metal-ligand compound wherein the metal M is W (tungsten) or Mo (molybdenum) on the support to form
a) either a metal-oxo compound wherein the metal is tethered to the support via one oxygen atom (precursor 1 of the supported metal complex 1), and/or
b) a metal-oxo compound wherein the metal is tethered to the support via two oxygen atoms (precursor 2 of the supported metal complex 2);
2. Alkylation of the compound obtained in step 1 to form
a) either a trialkyl-metal-oxo compound wherein the metal is tethered to the support via one oxygen atom, and/or
b) a dialkyl-metal-oxo compound wherein the metal is tethered to the silica via two oxygen atoms.

7. Process for the preparation of the supported metal compound according to the preceding claim wherein the grafting oxo-metal-ligand compound has a general formula MOX4 wherein M is W (tungsten) or Mo (molybdenum); X is any of F, Cl, Br, NRR' and OR (wherein R, R' is an alkyl and/or an aryl).

8. Process according to the preceding claim wherein the grafting oxo-metal-ligand MOX4 is selected from WOCl4, MoOCl4, or a mixture thereof.

9. Process according to any of claims 6 to 8 wherein the alkylation compound has the general formulae SnR4 and/or SnR3X (with R = alkyl, benzyl derivatives bearing at least one proton in the α position (preferably substituted benzyl ligands); and X is F, Cl, Br, I, OR, NRR' (wherein R, R' is an alkyl and/or an aryl)).

10. Process according to the preceding claim wherein the alkylation compound is tetramethyl-tin.

11. Process for metathesis or hydrogenolysis reactions of hydrocarbon compounds, more particularly for olefin metathesis reactions, in the presence of a supported metal compound catalyst according to any of claims 1 to 6.

12. Process for producing neohexene and/or 2,3-dimethylbutenes from the reaction of isobutene in the presence of a supported metal compound catalyst according to any of claims 1 to 6.

13. Process for controlling the ratios of neohexene to 2,3-dimethylbutenes during the reaction of isobutene in the presence of a supported metal compound catalyst according to any of claims 1 to 6.
